# EUROPEAN PATENT SPECIFICATION

(11) **EP 4 041 163 B1**
(45) Date of publication and mention of the grant of the patent: **30.10.2024**
(21) Application number: 20786058.6
(22) Date of filing: 22.09.2020
(51) Int. Cl.: A61F 13/02, A61M 25/02, A61F 13/00

(54) **TUBE SECUREMENT SYSTEM**
ROHRFIXIERSYSTEM
SYSTÈME DE FIXATION DE TUBULURE

(30) Priority: 11.10.2019 US 201962913796 P
(43) Date of publication of application: 17.08.2022
(73) Proprietor: Solventum Intellectual Properties Company, Maplewood, MN 55144 (US)
(72) Inventor: SIERACKI, James M., Saint Paul, Minnesota 55133-3427 (US); SCHEIBEL, Krystal J., Saint Paul, Minnesota 55133-3427 (US)
(74) Representative: Mathys & Squire
(86) International application number: PCT/IB2020/058843
(87) International publication number: WO 2021/069995

(56) References cited:
- EP-A1- 2 269 548
- WO-A1-2007/118636
- US-A1- 2005 020 957
- US-B1- 6 607 799

## Description

### Technical Field

The present disclosure generally relates to a medical article securement systems and methods for securing medical articles to the body of a patient, and particularly, for securing various catheter system, tubes, or other elongated devices to the body of a patient.

### Background

Tubes and catheters are inserted through a patient's skin to either introduce fluids to the patient or to remove fluids from the patient. The tubes must be secured to prevent the tube from slipping out of the patient. Commonly, surgical tape or sutures are used to hold tubing to the patient's skin.

One very common type of tube is an intravenous (IV) catheter. An IV catheter is inserted into the patient's bloodstream. Movement of an IV catheter while the catheter is inserted in a vein is a leading cause of catheter failure. When a catheter moves in a vein, it scrapes and pokes the inner wall of the vein, thereby irritating the vein. Repeated movement of the catheter can cause sufficient irritation of the vein to require that the catheter be removed and then a new catheter to be inserted in a different location along the same vein or in an entirely different vein. Therefore, a need exists for effective securement systems for tubes, such as catheters.

EP 2 269 548 A1 discloses an assembly containing at least two adhesive tapes with a U or V shape notch, or a slit that enables them to be placed one opposite the other and the V or U shape or the slit can be placed around an object extruding from a skin exit site minimizing the distance from the base of the U or V or the bottom slit of each adhesive.

### Summary

The invention is defined by the independent claims.

The disclosed tape system comprises a first tape and second tape each with an adhesive that interacts to secure a device such as tubing. The first tape and second tape each have a longitudinally extending midsection and laterally extending legs. The first tape overlaps the device, while the second tape is under the device, but with the legs of the second tape overlapping the legs of the first tape. This overlapping connection of the first tape and second tape strongly secures the tubing.

In one embodiment, the tape system includes first and second tapes, each tape having a first major surface comprising an adhesive, and a second major surface opposite the first major surface. The first and second tapes each have a midsection, and first and second legs extending from first and second ends of the midsection, the two legs extending in a direction lateral to the midsection. The first tape is applied over the second tape such that the first lateral direction of the first tape is opposed to the first lateral direction of the second tape. The midsections of the first and second tapes are aligned, but spaced apart from each other.

In a further embodiment, the second tape is applied over the first tape with the first leg of the second tape applied over the second leg of the first tape, and with the second leg of the second tape applied over the first leg of the first tape.

In one embodiment, the tape system includes a dressing overlying a device and a substrate. At least a portion of the tape system can be adhered to the dressing. At least a portion of the tape system can be adhered to the substrate. In some embodiments, the substrate is skin.

In one embodiment, a method for securing a device to a substrate includes applying the tape system to the device, the adhesive at the midsection of the first tape overlying a portion of the device with the first leg and second leg of the first tape adjacent to the device and overlying the substrate, and applying the midsection of the second tape of claim 1 under a portion of the device with the adhesive towards the substrate. The first leg of the second tape is overlying the second leg of the first tape, the second leg of the second tape is overlying the first leg of the first tape, and the device extends between the midsection of the first tape and the midsection of the second tape.

In a further embodiment, the method includes applying a dressing over the device, and under at least a portion of the tape system.

In some embodiments, the substrate is skin.

In one embodiment, a kit is provided that includes: 1) a dressing system and a first tape, the entire first tape overlying the dressing film; and 2) a second tape. The first and second tapes include features of the first and second tapes in embodiments of the tape system.

### Brief Description of Drawings

FIG. 1 is a first embodiment of a tape;
FIG. 2 is a side sectional view of FIG. 1 through line 2-2;
FIG. 3 is an alternate embodiment of a tape;
FIG. 4 is an embodiment of a tape system securing a tube in place;
FIG. 5 is the tape system of FIG. 4 with an underlying dressing;

While the above-identified drawings and figures set forth embodiments of the invention, other embodiments are also contemplated, as noted in the discussion. In all cases, this disclosure presents the invention by way of representation and not limitation. Other modifications and embodiments can be devised by those skilled in the art, which fall within the scope of this invention. The figures may not be drawn to scale.

### Detailed Description

### Tape System

The disclosed tape system 1 comprises a first tape 100 and second tape 200 each with an adhesive that interact to secure a device such as tubing. The first tape and second tape each have a longitudinally extending midsection and laterally extending legs. The first tape overlaps the device, while the second tape is under the device, but with the legs of the second tape overlapping the legs of the first tape. This overlapping connection of the first tape and second tape strongly secures the tubing.

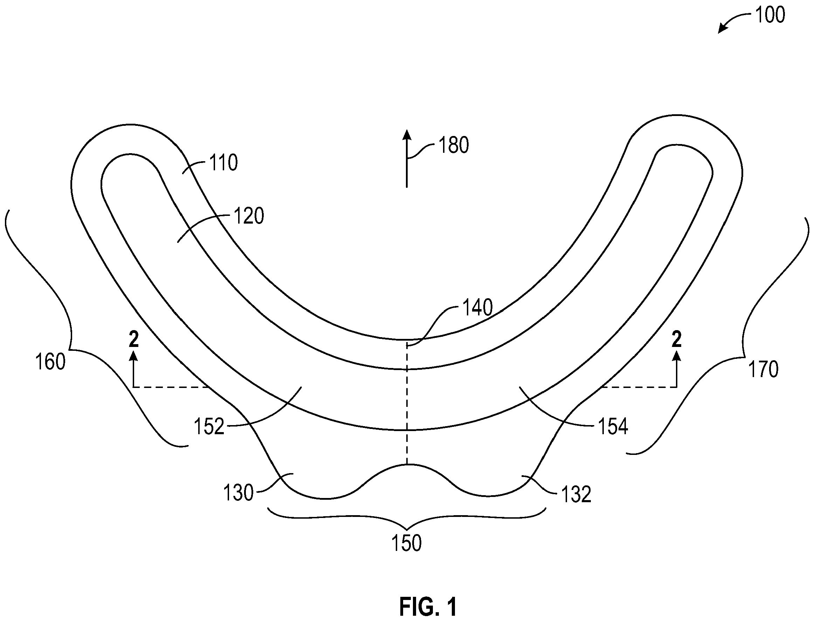

FIG. 1 is an embodiment of the first tape 100 having a backing 110. Backing 110 has a first major surface and a second major surface opposite the first major surface. The first major surface of backing 110 has an adhesive. Tape 100 has midsection 150, the midsection having a first end 152 and a second end 154. A first leg 160 extends away from midsection 150 at first end 152 in a lateral direction 180, and a second leg 170 extends away from midsection 150 at second end 154 in the lateral direction 180. Midsection 150 extends in a longitudinal direction perpendicular to lateral direction 180. In this embodiment, the midsection 150 is curved with the first leg 160 and second leg 170 extending along the same curved shape forming a "C" shaped tape 100. In this embodiment, the tape 100 is symmetrical. Other shapes and configurations of the tape 100 can be used such as being asymmetrical, having wider and narrower portions, legs of differing lengths, straight, curved or a combination for the arrangements of the midsection and legs, so long as the legs extend in the same lateral direction.

Optionally, midsection 150 has tabs 130 and 132 extending away from midsection 150. Midsection 150 optionally includes a perforation set 140 extending laterally across midsection 150. Optional tabs 130 and 132, and optional perforation set 140 can be beneficial in removal of the tape system.

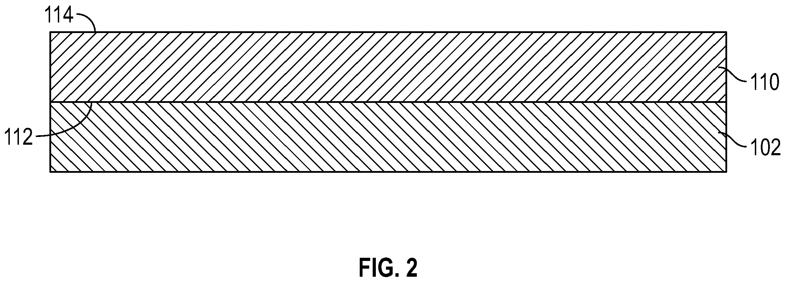

FIG. 2 show a side sectional view of tape 100, showing backing 110 as a layer having a first major surface 112 and a second major surface 114 opposed to first major surface 112. First major surface 112 of backing 110 has a layer of adhesive 102. In some embodiments, the adhesive is a skin-adhesive.

In some embodiments, backing 110 can include multiple layers, including, for example, a reinforcement layer 120 to provide stiffness and/or strength to tape 100 for securing a device, such as a tube, or more specifically a catheter. In some embodiments, reinforcement layer 120 is disposed at second major surface 114. The reinforcement layer may be visually clear or have a color to indicate its presence.

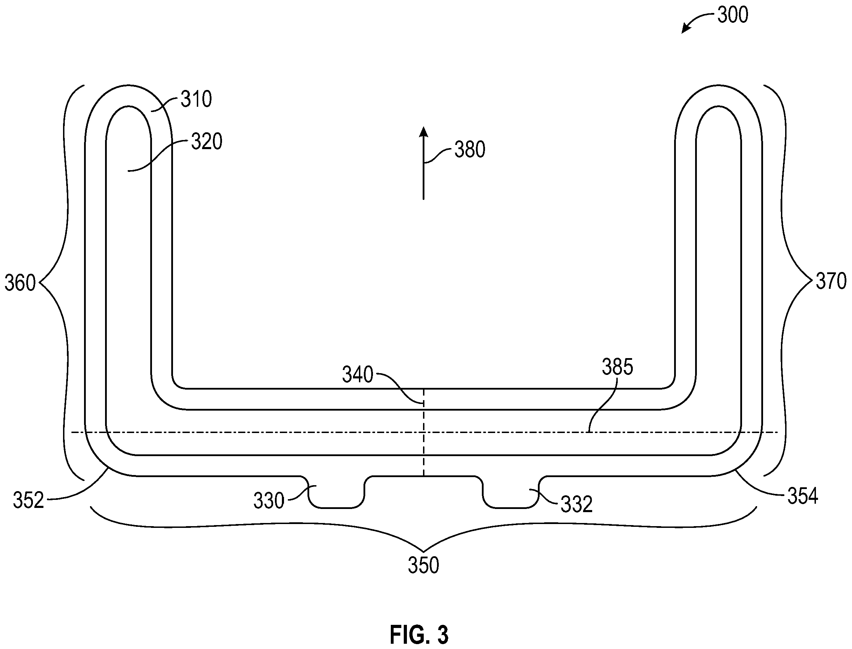

FIG. 3 illustrates an alternate embodiment of a tape 300 having a backing 310. In contrast the "C" shape of tape 100, tape 300 has a more rectilinear shape. Backing 310 has a first major surface and a second major surface opposite the first major surface. The first major surface of backing 310 has an adhesive. Tape 300 has midsection 350 extending in a longitudinal direction along axis 385, the midsection having a first end 352 and a second end 354. A first leg 360 extends away from midsection 350 at first end 352 in a lateral direction 380, and a second leg 370 extends away from midsection 350 at second end 354 in the lateral direction 380. The longitudinal direction is perpendicular to the lateral direction 380. In this embodiment, the tape 300 is symmetrical. Other shape and configurations of the tape 300 can be used such as being asymmetrical, having wider and narrower portions, legs of differing lengths, straight, curved or a combination for the arrangements of the midsection and legs, so long as the legs extend in the same lateral direction.

Optionally, midsection 350 has tabs 330 and 332 extending away from midsection 350. Midsection 350 optionally includes a perforation set 340 extending laterally across midsection 350. Optional tabs 330 and 332, and optional perforation set 340 can be beneficial in removal of the tape system.

In some embodiments, backing 310 can include multiple layers, including, for example, a reinforcement layer 320 to provide stiffness and/or strength to tape 300 for securing a device, such as a tube, or more specifically a catheter. In some embodiments, reinforcement layer 320 is disposed at the second major surface of backing 310.

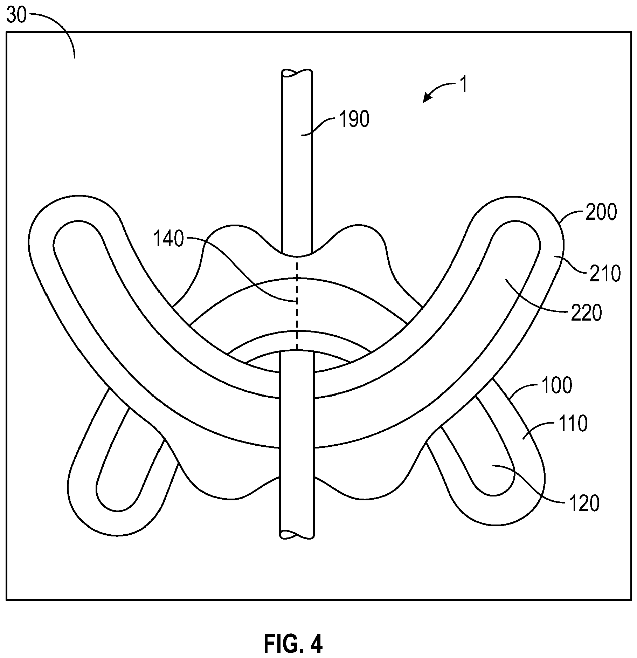

FIG. 4 shows the tape system 1 with the first tape 100 and second tape 200. Second tape 200 is substantially the same as first tape 100. Second tape 200 includes a midsection and a first leg and a second leg.

As shown in FIG. 4, first tape 100 is over tube 190 and overlies a substrate 30. Second tape 200 is under the tube 190, but with the first leg and the second leg of the second tape 200 placed over the first leg and the second leg of tape 100. The midsections of tapes 100 and 200 are aligned, but spaced apart, to allow, for example, tube 190 to be situated between the midsections of tapes 100 and 200. In this configuration, tape system 1 can advantageously secure tube 190 from being pulled away from the substrate.

In some embodiments, tapes 100 and 200 in tape system 1 are identical. A significant advantage of having tapes 100 and 200 be identical is simplification of applying the tape, in part by eliminating the need to select among two different types of tape during a catheterization process, where speed and ease of apply the tape system is highly valuable, to both the patient and the medical professional performing the catheterization. In some situations, only one type of the tape needs to be available to form the tape system, which can result in additional benefits in the areas of packaging, inventory, and cost.

In some other embodiments, tapes 100 and 200 in tape system 1 are not identical; for example, tapes 100 and 200 may differ from each other in shape, size, materials, in whether or not a perforation set is present, among other possible differences, or combinations of such differences. A tape system that uses non-identical tapes could be a useful, for example, in cases where the skin surface available for application of the tape system has restricted geometric requirements.

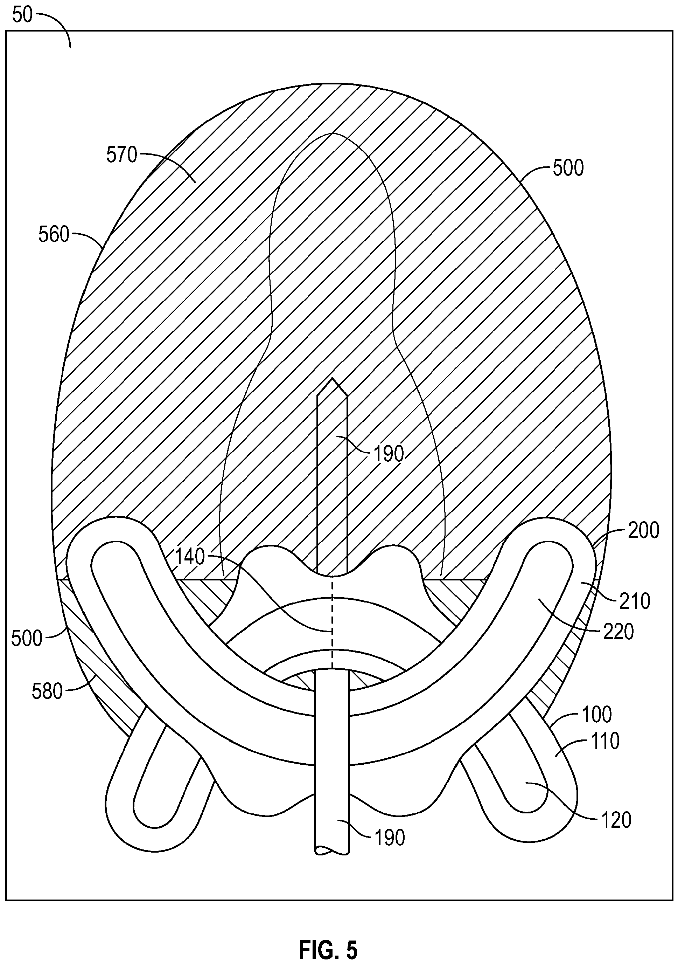

FIG. 5 shows a dressing system including the tape system 1 described above in FIG. 4 and an underlying dressing 500. Dressing 500 has a body layer 570 defined by a perimeter 560, body layer 570 having a first major surface opposite a second major surface. The second major surface has an adhesive. In the embodiment shown, a portion of body layer 570 includes a support layer 580. Dressing 500 overlies tube 190 and substrate 50. In embodiments where tube 190 is an IV catheter inserted into a vein, the dressing 500 can beneficially provide a physical protection of the insertion site from fluids, particulates, microorganisms, and the like. In the embodiment shown, a portion of tape system 1 is adhered to the first major surface of the dressing 500, and a portion of tape system 1 is adhered to substrate 50.

### Backing Material

The backing layer 110 can be any material that provides mechanical stiffening of the tape while also allowing enough flexibility to allow for conformability and comfortable wear. Backing material can be a film, a paper, or a fabric layer material, such as a woven, knitted, or nonwoven fabric. Backing material can be elastic or stiff. In some embodiments, the backing layer material is a woven, knitted, or nonwoven material. One example of a nonwoven material is a high strength nonwoven fabric available from E. I. Dupont de Nemours & Company of Wilmington, Delaware, under the trademark SONTARA. Other suitable nonwoven webs include a hydroentangled polyester fabric available from Vertac, a division of International Paper of Walpole, MN. Another suitable nonwoven web is the nonwoven elastomeric web described in U.S. Pat. 5,230,701. The backing can be a high moisture vapor permeable film backing. U.S. Pat. No. 3,645,835 describes methods of making such films and methods for testing their permeability.

The backing may be a single or multilayer construction. In some embodiments, an optional reinforcing layer material may be used with the backing. The reinforcing material may be as pliable as a thick adhesive or as stiff as a solid material (e.g., a paper or a film). For example, U.S. Pat. No. 5,088,483 discloses a permanent adhesive as a reinforcement material.

### Adhesive

Any number of adhesives can be used with the tape and on the dressing, if included. Suitable adhesives are pressure sensitive and in certain embodiments have a relatively high moisture vapor transmission rate to allow for moisture evaporation. Suitable pressure sensitive adhesives include those based on acrylates, urethane, hydrogels, hydrocolloids, block copolymers, silicones, rubber-based adhesives (including natural rubber, polyisoprene, polyisobutylene, butyl rubber etc.) as well as combinations of these adhesives. The adhesive component may contain tackifiers, plasticizers, rheology modifiers as well as active components, for example an antimicrobial agent.

The pressure sensitive adhesive is usually reasonably skin compatible and "hypoallergenic", such as the acrylate copolymers described in U.S. Pat. No. RE 24,906. Particularly useful is a 97:3 isooctyl acrylate: acrylamide copolymer, as is 70:15:15 isooctyl acrylate: ethyleneoxide acrylate: acrylic acid terpolymer described in U.S. Pat. No. 4,737,410. Additional useful adhesives are described in U.S. Pat. Nos. 3,389,827; 4,112,213; 4,310,509; and 4,323,557. Inclusion of medicaments or antimicrobial agents in the adhesive is also contemplated, as described in U.S. Pat. Nos. 4,310,509 and 4,323,557.

Silicone adhesives can also be used. Generally, silicone adhesives can provide suitable adhesion to skin while gently removing from skin. Suitable silicone adhesives are disclosed in PCT Publications WO2010/056541 and WO2010/056543.

Adhesive layers of the present disclosure can be flood-coated, or pattern-coated. The adhesive layer is preferably pattern-coated to allow for better vapor transmission. An example of useful pattern coating is described in U.S. Patent No. 4,595,001.

### Dressing

In some embodiments, suitable dressings of the present disclosure include a thin, flexible and transparent polymeric film body layer. In some embodiments, the dressing further includes a support material. In general, the support layer materials can include, but are not limited to, an elastic film, a non-elastic film, non-woven fibrous web, woven fibrous web, knits, and polyethylene/vinyl acetate copolymer-coated papers and polyester films. Examples of suitable dressings can be found, for example, in WO2019/073326, U.S. Patent Application Publication No. US2016/0015570, and in U.S. Provisional Patent Application No. 62/827,400 "A Conformable Dressing" (filed April 1, 2019). One example of a commercially available medical dressing is TEGADERM IV ADVANCED DRESSING (3M Co., St. Paul, MN).

### Release Liner

In some embodiments, tapes and dressings of the present disclosure include a release liner film disposed on the skin adhesive layer (for example, adhesive layer 202 in FIG. 2). Release liners can be made of kraft papers, polyethylene, polypropylene, polyester or composites of any of these materials. The films are preferably coated with release agents such as fluorochemicals or silicones. For example, U.S. Pat. No. 4,472,480 describes low surface energy perfluorochemical liners. The liners are papers, polyolefin films, or polyester films coated with silicone release materials. Examples of commercially available silicone coated release papers are POLYSLIK^{™}, silicone release papers available from Rexam Release (Bedford Park, Ill.) and silicone release papers supplied by Loparex Group (Willowbrook, Ill.).

### Kit

In some embodiments, a kit is provided that includes: 1) a dressing having a first major surface, a second major surface opposite the first major surface, defined by a perimeter around a body layer, the second major surface comprising a skin-compatible adhesive, with a first tape of the disclosure overlying the first major surface of the dressing; and 2) a second tape of the disclosure. In some embodiments, the entire first surface of the first tape overlies first major surface of the dressing. In some embodiments, a portion of the body of the dressing includes a support layer, and the entire first surface of the first tape overlies the support layer.

The kit can facilitate applying the dressing and first tape together over a device inserted into a substrate, and applying the second tape under the device, with the first leg of the second tape applied over the second leg of the first tape, and with the second leg of the second tape applied over the first leg of the first tape, with the device extending between the midsection of the first tape and midsection of the second tape. In some embodiments, the first and second components of the kit can be disposed on a single release liner, to simplify application of the components to a patient.

Each embodiment shown in the figures is illustrated as a separate embodiment for clarity in illustrating a variety of features of the tape systems and dressing systems of the present disclosure. However, it should be understood that any combination of elements and features of any of the embodiments illustrated in the figures and described herein can be employed in the tape systems and dressing systems of the present disclosure.

## Claims

1. A dressing system comprising
a tape system (1) comprising
a first tape (100; 300) comprising:
a first major surface (112) with a first tape adhesive (102) and a second major surface (114) opposite the first major surface;
a midsection (150; 350) extending in a longitudinal direction (385), the midsection having a first end (152; 352) and a second end (154; 354);
a first leg (160; 360) extending from the midsection at the first end in a first lateral direction (180; 380);
a second leg (170; 370) extending from the midsection at the second end in the first lateral direction; and
a second tape (200) comprising:
a first major surface with a second tape adhesive and a second major surface opposite the first major surface;
a midsection extending in a longitudinal direction, the midsection having a first end and a second end;
a first leg extending from the midsection at the first end in a first lateral direction;
a second leg extending from the midsection at the second end in the first lateral direction;
wherein the second tape is applied over the first tape with the first lateral direction of the first tape opposed to the first lateral direction of the second tape; and
wherein the midsection of the first tape is aligned with but spaced apart from the midsection of the second tape,
wherein the dressing system further comprises a dressing (500) comprising
a first major surface, a second major surface opposite the first major surface, defined by a perimeter (560) around a body layer (570), the second major surface comprising an adhesive; and
wherein at least a portion of the tape system overlies the first major surface of the dressing.

2. The dressing system of claim 1, wherein the longitudinal and first lateral directions (180; 385, 380) in the first tape (100, 300) are perpendicular to each other.

3. The dressing system of claim 1, wherein either the midsection (350) of the first tape (300) extends in a straight line or the midsection (150) of the first tape (100) is curved.

4. The dressing system of claim 1, wherein the first tape (100, 300) has a perforation set (140; 340) extending laterally across the midsection (150, 350).

5. The dressing system of any one of claims 1 to 4, wherein the second tape (200) is applied over the first tape (100, 300) with the first leg of the second tape applied over the second leg (170; 370) of the first tape, and with the second leg of the second tape applied over the first leg (160; 360) of the first tape.

6. The dressing system of any one of claims 1 to 5, wherein at least one of the first tape (100, 300) and second tape (200) comprises a reinforcement layer (120, 220; 320).

7. The dressing system of any one of claims 1 to 6, wherein the first tape (100, 300) and the second tape (200) are identical.

8. The dressing system of any one of claims 1 to 7, wherein a device (190) extends between the midsection (150; 350) of the first tape (100; 300) and midsection of the second tape (200).

9. The dressing system of claim 8, wherein the midsection (150; 350) of the first tape (100; 300) overlaps the device (190) and the midsection of the second tape (200) is under the device.

10. The dressing system of any one of claims 1 to 9, wherein the entire first major surface (112) of the first tape (100; 300) overlies the first major surface of the dressing (500).

11. The dressing system of any of claims 1 to 10, wherein the second surface of the dressing (500) overlies a substrate (50), and at least a portion of the tape system (1) overlies the substrate.

12. A method of securing a device (190) to a substrate (50), the method comprising:
applying the dressing (500) of claim 1 over a portion of the device,
applying the first tape adhesive (102) at the midsection (150; 350) of the first tape (100; 300) of claim 1 over a portion of the device with the first leg (160; 360) and second leg (170; 370) of the first tape adjacent to the device and overlying the substrate;
applying the midsection of the second tape (200) of claim 1 under a portion of the device with the second tape adhesive towards the substrate, wherein the first leg of the second tape overlies the second leg of the first tape, wherein the second leg of the second tape overlies the first leg of the first tape, and wherein the device extends between the midsection of the first tape and the midsection of the second tape.

13. The method of claim 12, wherein the dressing (500) is applied over the portion of the device (190) prior to the applications of the first and second tapes (100, 200; 300).

14. A kit comprising:
1) a dressing (500) comprising:
a first major surface, a second major surface opposite the first major surface,
defined by a perimeter (560) around a body layer (570), the second major surface comprising an adhesive; and
a first tape (100; 300) comprising:
a first major surface (112) with a first tape adhesive (102) and a second major surface (114) opposite the first major surface;
a midsection (150; 350) extending in a longitudinal direction (385), the midsection having a first end (152; 352) and a second end (154; 354);
a first leg (160; 360) extending from the midsection at the first end in a first lateral direction (180; 380);
a second leg (170; 370) extending from the midsection at the second end in the first lateral direction;
wherein the first tape overlies the first major surface of the dressing; and
2) a second tape (200) comprising:
a first major surface with a second tape adhesive and a second major surface opposite the first major surface;
a midsection extending in a longitudinal direction, the midsection having a first end and a second end;
a first leg extending from the midsection at the first end in a first lateral direction; and
a second leg extending from the midsection at the second end in the first lateral direction.

15. A kit of claim 14, wherein the entire first major surface (112) of first tape (100; 300) overlies the first major surface of the dressing (500).

## Patentansprüche

1. Ein Verbandsystem, aufweisend
ein Bandsystem (1), aufweisend
ein erstes Band (100; 300), aufweisend:
eine erste Hauptoberfläche (112) mit einem Klebstoff (102) des ersten Bands und eine zweite Hauptoberfläche (114) gegenüberliegend der ersten Hauptoberfläche;
einen Mittelabschnitt (150; 350), der sich in einer Längsrichtung (385) erstreckt, wobei der Mittelabschnitt ein erstes Ende (152; 352) und ein zweites Ende (154; 354) hat;
einen ersten Schenkel (160; 360), der sich von dem Mittelabschnitt an dem ersten Ende in einer ersten seitlichen Richtung (180; 380) erstreckt;
einen zweiten Schenkel (170; 370), der sich von dem Mittelabschnitt an dem zweiten Ende in der ersten seitlichen Richtung erstreckt; und
ein zweites Band (200), aufweisend:
eine erste Hauptoberfläche mit einem Klebstoff des zweiten Bands und eine zweite Hauptoberfläche gegenüberliegend der ersten Hauptoberfläche;
einen Mittelabschnitt, der sich in einer Längsrichtung erstreckt, wobei der Mittelabschnitt ein erstes Ende und ein zweites Ende hat;
einen ersten Schenkel, der sich von dem Mittelabschnitt an dem ersten Ende in einer ersten seitlichen Richtung erstreckt;
einen zweiten Schenkel, der sich von dem Mittelabschnitt an dem zweiten Ende in der ersten seitlichen Richtung erstreckt;
wobei das zweite Band über dem ersten Band angebracht wird, wobei die erste seitliche Richtung des ersten Bands der ersten seitlichen Richtung des zweiten Bands gegenüberliegt; und
wobei der Mittelabschnitt des ersten Bands mit dem Mittelabschnitt des zweiten Bands ausgerichtet, jedoch von diesem beabstandet ist,
wobei das Verbandsystem ferner einen Verband (500) aufweist, aufweisend
eine erste Hauptoberfläche, eine zweite Hauptoberfläche gegenüberliegend der ersten Hauptoberfläche, definiert durch einen Umfang (560) um eine Körperschicht (570) herum, die zweite Hauptoberfläche aufweisend einen Klebstoff; und
wobei mindestens ein Teil des Bandsystems die erste Hauptoberfläche des Verbands überlagert.

2. Das Verbandsystem nach Anspruch 1, wobei die Längsrichtung und die erste seitliche Richtung (180; 385, 380) in dem ersten Band (100, 300) senkrecht zueinander sind.

3. Das Verbandsystem nach Anspruch 1, wobei sich entweder der Mittelabschnitt (350) des ersten Bands (300) in einer geraden Linie erstreckt oder der Mittelabschnitt (150) des ersten Bands (100) gekrümmt ist.

4. Das Verbandsystem nach Anspruch 1, wobei das erste Band (100, 300) einen Perforationssatz (140; 340) hat, der sich seitlich quer über den Mittelabschnitt (150, 350) erstreckt.

5. Das Verbandsystem nach einem der Ansprüche 1 bis 4, wobei das zweite Band (200) über dem ersten Band (100, 300) angebracht wird, wobei der erste Schenkel des zweiten Bands über dem zweiten Schenkel (170; 370) des ersten Bands angebracht wird und wobei der zweite Schenkel des zweiten Bands über dem ersten Schenkel (160; 360) des ersten Bands angebracht wird.

6. Das Verbandsystem nach einem der Ansprüche 1 bis 5, wobei mindestens eines des ersten Bands (100, 300) und des zweiten Bands (200) eine Verstärkungsschicht (120, 220; 320) aufweist.

7. Das Verbandsystem nach einem der Ansprüche 1 bis 6, wobei das erste Band (100, 300) und das zweite Band (200) identisch sind.

8. Das Verbandsystem nach einem der Ansprüche 1 bis 7, wobei sich eine Vorrichtung (190) zwischen dem Mittelabschnitt (150; 350) des ersten Bands (100; 300) und dem Mittelabschnitt des zweiten Bands (200) erstreckt.

9. Das Verbandsystem nach Anspruch 8, wobei der Mittelabschnitt (150; 350) des ersten Bands (100; 300) die Vorrichtung (190) überlappt und der Mittelabschnitt des zweiten Bands (200) unter der Vorrichtung ist.

10. Das Verbandsystem nach einem der Ansprüche 1 bis 9, wobei die gesamte erste Hauptoberfläche (112) des ersten Bands (100; 300) die erste Hauptoberfläche des Verbands (500) überlagert.

11. Das Verbandsystem nach einem der Ansprüche 1 bis 10, wobei die zweite Oberfläche des Verbands (500) ein Substrat (50) überlagert und mindestens ein Teil des Bandsystems (1) das Substrat überlagert.

12. Ein Verfahren zum Befestigen einer Vorrichtung (190) an einem Substrat (50), das Verfahren aufweisend:
Anbringen des Verbands (500) nach Anspruch 1 über einem Teil der Vorrichtung,
Anbringen des Klebstoffs (102) des ersten Bands an dem Mittelabschnitt (150; 350) des ersten Bands (100; 300) nach Anspruch 1 über einem Teil der Vorrichtung, wobei der erste Schenkel (160; 360) und der zweite Schenkel (170; 370) des ersten Bands an die Vorrichtung angrenzen und das Substrat überlagern;
Anbringen des Mittelabschnitts des zweiten Bands (200) nach Anspruch 1 unter einem Teil der Vorrichtung mit dem Klebstoff des zweiten Bands zu dem Substrat hin, wobei der erste Schenkel des zweiten Bands den zweiten Schenkel des ersten Bands überlagert, wobei der zweite Schenkel des zweiten Bands den ersten Schenkel des ersten Bands überlagert und wobei sich die Vorrichtung zwischen dem Mittelabschnitt des ersten Bands und dem Mittelabschnitt des zweiten Bandes erstreckt.

13. Das Verfahren nach Anspruch 12, wobei der Verband (500) vor den Anbringungen des ersten und des zweiten Bands (100, 200; 300) über dem Teil der Vorrichtung (190) angebracht wird.

14. Ein Kit, aufweisend:
1) einen Verband (500), aufweisend:
eine erste Hauptoberfläche, eine zweite Hauptoberfläche gegenüberliegend der ersten Hauptoberfläche, definiert durch einen Umfang (560) um eine Körperschicht (570) herum, die zweite Hauptoberfläche aufweisend einen Klebstoff; und
ein erstes Band (100; 300), aufweisend:
eine erste Hauptoberfläche (112) mit einem Klebstoff (102) des ersten Bands und eine zweite Hauptoberfläche (114) gegenüberliegend der ersten Hauptoberfläche;
einen Mittelabschnitt (150; 350), der sich in einer Längsrichtung (385) erstreckt, wobei der Mittelabschnitt ein erstes Ende (152; 352) und ein zweites Ende (154; 354) hat;
einen ersten Schenkel (160; 360), der sich von dem Mittelabschnitt an dem ersten Ende in einer ersten seitlichen Richtung (180; 380) erstreckt;
einen zweiten Schenkel (170; 370), der sich von dem Mittelabschnitt an dem zweiten Ende in der ersten seitlichen Richtung erstreckt;
wobei das erste Band die erste Hauptoberfläche des Verbands überlagert; und
2) ein zweites Band (200), aufweisend:
eine erste Hauptoberfläche mit einem Klebstoff des zweiten Bands und eine zweite Hauptoberfläche gegenüberliegend der ersten Hauptoberfläche;
einen Mittelabschnitt, der sich in einer Längsrichtung erstreckt, wobei der Mittelabschnitt ein erstes Ende und ein zweites Ende hat;
einen ersten Schenkel, der sich von dem Mittelabschnitt an dem ersten Ende in einer ersten seitlichen Richtung erstreckt; und einen zweiten Schenkel, der sich von dem Mittelabschnitt an dem zweiten Ende in der ersten seitlichen Richtung erstreckt.

15. Ein Kit nach Anspruch 14, wobei die gesamte erste Hauptoberfläche (112) des ersten Bands (100; 300) die erste Hauptoberfläche des Verbands (500) überlagert.

## Revendications

1. Système de pansement comprenant
un système de bande (1) comprenant
une première bande (100 ; 300) comprenant :
une première surface principale (112) avec une première bande adhésive (102) et une seconde surface principale (114) opposée à la première surface principale ;
une section médiane (150 ; 350) s'étendant dans une direction longitudinale (385), la section médiane ayant une première extrémité (152 ; 352) et une seconde extrémité (154 ; 354) ;
une première patte (160 ; 360) s'étendant à partir de la section médiane au niveau de la première extrémité dans une première direction latérale (180 ; 380) ;
une seconde patte (170 ; 370) s'étendant à partir de la section médiane au niveau de la seconde extrémité dans la première direction latérale ; et
une seconde bande (200) comprenant :
une première surface principale avec une seconde bande adhésive et une seconde surface principale opposée à la première surface principale ;
une section médiane s'étendant dans une direction longitudinale, la section médiane ayant une première extrémité et une seconde extrémité ;
une première patte s'étendant à partir de la section médiane au niveau de la première extrémité dans une première direction latérale ;
une seconde patte s'étendant à partir de la section médiane au niveau de la seconde extrémité dans la première direction latérale ;
dans lequel la seconde bande est appliquée sur la première bande avec la première direction latérale de la première bande opposée à la première direction latérale de la seconde bande ; et
dans lequel la section médiane de la première bande est alignée avec la section médiane de la seconde bande, mais en est éloignée,
dans lequel le système de pansement comprend en outre un pansement (500) comprenant
une première surface principale, une seconde surface principale opposée à la première surface principale, définie par un périmètre (560) autour d'une couche de corps (570), la seconde surface principale comprenant un adhésif ; et
dans lequel au moins une partie du système de bande recouvre la première surface principale du pansement.

2. Système de pansement selon la revendication 1, dans lequel la direction longitudinale et les premières directions latérales (180 ; 385, 380) de la première bande (100, 300) sont perpendiculaires les unes aux autres.

3. Système de pansement selon la revendication 1, dans lequel la section médiane (350) de la première bande (300) s'étend en ligne droite ou la section médiane (150) de la première bande (100) est incurvée.

4. Système de pansement selon la revendication 1, dans lequel la première bande (100, 300) a un ensemble de perforations (140 ; 340) s'étendant latéralement à travers la section médiane (150, 350).

5. Système de pansement selon l'une quelconque des revendications 1 à 4, dans lequel la seconde bande (200) est appliquée sur la première bande (100, 300), la première patte de la seconde bande étant appliquée sur la seconde patte (170 ; 370) de la première bande, et la seconde patte de la seconde bande étant appliquée sur la première patte (160 ; 360) de la première bande.

6. Système de pansement selon l'une quelconque des revendications 1 à 5, dans lequel au moins l'une parmi la première bande (100, 300) et la seconde bande (200) comprend une couche de renforcement (120, 220 ; 320).

7. Système de pansement selon l'une quelconque des revendications 1 à 6, dans lequel la première bande (100, 300) et la seconde bande (200) sont identiques.

8. Système de pansement selon l'une quelconque des revendications 1 à 7, dans lequel un dispositif (190) s'étend entre la section médiane (150 ; 350) de la première bande (100 ; 300) et la section médiane de la seconde bande (200).

9. Système de pansement selon la revendication 8, dans lequel la section médiane (150 ; 350) de la première bande (100 ; 300) chevauche le dispositif (190) et la section médiane de la seconde bande (200) est sous le dispositif.

10. Système de pansement selon l'une quelconque des revendications 1 à 9, dans lequel toute la première surface principale (112) de la première bande (100 ; 300) recouvre la première surface principale du pansement (500).

11. Système de pansement selon l'une quelconque des revendications 1 à 10, dans lequel la seconde surface du pansement (500) recouvre un substrat (50), et au moins une partie du système de bande (1) recouvre le substrat.

12. Procédé de fixation d'un dispositif (190) sur un substrat (50), le procédé comprenant :
l'application du pansement (500) selon la revendication 1 sur une partie du dispositif,
l'application de la première bande adhésive (102) au niveau de la section médiane (150 ; 350) de la première bande (100 ; 300) selon la revendication 1 sur une partie du dispositif avec la première patte (160 ; 360) et la seconde patte (170 ; 370) de la première bande adjacentes au dispositif et recouvrant le substrat ;
l'application de la section médiane de la seconde bande (200) selon la revendication 1 sous une partie du dispositif avec la seconde bande adhésive vers le substrat, dans lequel la première patte de la seconde bande recouvre la seconde patte de la première bande, dans lequel la seconde patte de la seconde bande recouvre la première patte de la première bande, et dans lequel le dispositif s'étend entre la section médiane de la première bande et la section médiane de la seconde bande.

13. Procédé selon la revendication 12, dans lequel le pansement (500) est appliqué sur la partie du dispositif (190) avant les applications des première et seconde bandes (100, 200 ; 300).

14. Kit comprenant :
1) un pansement (500), comprenant :
une première surface principale, une seconde surface principale opposée à la première surface principale, définie par un périmètre (560) autour d'une couche de corps (570), la seconde surface principale comprenant un adhésif ; et
une première bande (100 ; 300) comprenant :
une première surface principale (112) avec une première bande adhésive (102) et une seconde surface principale (114) opposée à la première surface principale ;
une section médiane (150 ; 350) s'étendant dans une direction longitudinale (385), la section médiane ayant une première extrémité (152 ; 352) et une seconde extrémité (154 ; 354) ;
une première patte (160 ; 360) s'étendant à partir de la section médiane au niveau de la première extrémité dans une première direction latérale (180 ; 380) ;
une seconde patte (170 ; 370) s'étendant à partir de la section médiane au niveau de la seconde extrémité dans la première direction latérale ;
dans lequel la première bande recouvre la première surface principale du pansement ; et
2) une seconde bande (200) comprenant :
une première surface principale avec une seconde bande adhésive et une seconde surface principale opposée à la première surface principale ;
une section médiane s'étendant dans une direction longitudinale, la section médiane ayant une première extrémité et une seconde extrémité ;
une première patte s'étendant à partir de la section médiane au niveau de la première extrémité dans une première direction latérale ; et une seconde patte s'étendant à partir de la partie médiane au niveau de la seconde extrémité dans la première direction latérale.

15. Kit selon la revendication 14, dans lequel toute la première surface principale (112) de la première bande (100 ; 300) recouvre la première surface principale du pansement (500).
